# EUROPÄISCHE PATENTSCHRIFT

(11) **EP 2 198 899 B1**
(45) Veröffentlichungstag und Bekanntmachung des Hinweises auf die Patenterteilung: **07.10.2015**
(21) Anmeldenummer: 09177440.6
(22) Anmeldetag: 30.11.2009
(51) Int. Cl.: A61L 31/18, A61L 31/02, A61L 31/10, A61L 31/14, C23C 16/00

(54) **Vorrichtung und Verfahren zur Herstellung derselben**
Device and method for producing same
Dispositif et son procédé de fabrication

(30) Priorität: 18.12.2008 DE 102008054845
(43) Veröffentlichungstag der Anmeldung: 23.06.2010
(73) Patentinhaber: Biotronik VI Patent AG, 6341 Baar (CH)
(72) Erfinder: Bayer, Ullrich, 18211, Admannshagen-Bargeshagen (DE); Lootz, Daniel, 18119, Rostock (DE)
(74) Vertreter: Randoll, Sören

(56) Entgegenhaltungen:
- EP-A1- 1 886 650
- EP-A2- 0 824 900
- EP-A2- 1 886 701
- EP-A2- 1 941 918
- DE-A1- 10 127 770
- US-A1- 2006 016 690
- US-A1- 2006 224 237
- US-A1- 2008 057 101
- US-A1- 2008 086 195

## Beschreibung

Die vorliegende Erfindung betrifft eine Vorrichtung, insbesondere für die medizinische Anwendung als Endoprothese, bevorzugt als intraluminale Endoprothese, mit einem Grundkörper, der zumindest teilweise aus einem metallischen Material besteht, und mit einem an dem Grundkörper befestigten Funktionselement, das zumindest in einem Teil seines Volumens eine verglichen mit dem Material des Grundkörpers verschiedene metallische Materialzusammensetzung aufweist, die mindestens teilweise radioopakes und/oder röntgenopakes Material aufweist, sowie ein Verfahren zur Herstellung einer solchen Vorrichtung.

Stents sind endovaskuläre Prothesen (Endoprothesen) oder Implantate, die zur Behandlung von Stenosen (Gefäßverengungen) eingesetzt werden können. Sie weisen einen Grundkörper in Form eines hohlzylinder- oder röhrenförmigen Grundgitters auf, das an beiden Längsenden der Röhren offen ist. Das röhrenförmige Grundgitter einer derartigen Endoprothese wird in das zu behandelnde Gefäß eingesetzt und dient dazu, das Gefäß zu stützen.

Derartige Stents oder andere Endoprothesen sowie Vorrichtungen, die beispielsweise im Flugzeugbau eingesetzt werden, weisen in ihrem Grundkörper häufig metallische Materialien auf. Hierbei können die metallischen Materialien einen biodegradierbaren Werkstoff bilden, wobei auch polymere, biodegradierbare Materialien enthalten sein können.

Unter Biodegradation werden hydrolytische, enzymatische und andere stoffwechselbedingte Abbauprozesse im lebenden Organismus verstanden, die vor allem durch die mit der Endoprothese in Kontakt gelangenden Körperflüssigkeiten verursacht werden und zu einer allmählichen Auflösung zumindest großer Teile der Endoprothese führen. Synonym zu dem Begriff Biodegradation wird häufig der Begriff Biokorrosion verwendet. Der Begriff Bioresorption umfasst die anschließende Resorption der Abbauprodukte durch den lebenden Organismus.

Für den Grundkörper biodegradierbarer Endoprothesen geeignete Werkstoffe (Grundmaterial) können auch aus mehreren Materialien bestehen. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Metallische biodegradierbare Werkstoffe basieren auf Legierungen von Magnesium, Eisen, Zink und/oder Wolfram.

Die vorliegende Erfindung bezieht sich auf Endoprothesen oder andere Vorrichtungen mit einem Grundkörper, dessen Material einen metallischen Werkstoff enthält. Hierbei kommen neben den genannten biodegradierbaren Materialien auch weitere metallische Werkstoffe in Frage.

Die Ermittlung der Position eines Stents oder anderer Vorrichtungen geschieht häufig mittels bildgebender Verfahren, beispielsweise mittels einer Röntgenstrahleinrichtung. Aufgrund der kleinen Ordnungszahl und der geringen Dichte des biodegradierbaren Materials Magnesium und seiner Legierungen ist die Röntgensichtbarkeit der daraus gefertigten medizinischen Implantate sehr gering. Um diesen Nachteil zu beheben, ist es bekannt, medizinische Vorrichtungen mit Funktionselementen zu versehen, die zumindest in einem Teil ihres Volumens eine verglichen mit dem Material des Grundkörpers verschiedene Materialzusammensetzung aufweisen. Diese sogenannten Marker oder Funktionselemente enthalten insbesondere ein Material, das die Röntgenstrahlen und/oder andere elektromagnetische Strahlen stärker absorbiert (im Folgenden als röntgenopakes oder radioopakes Material bezeichnet) als das Material des Grundkörpers.

In der Druckschrift US 6,355,058 B1 wird ein Stent beschrieben, bei dem radioopake Marker in einem polymeren Bindemittel als Partikel eingeschlossen sind. Der Binder ist auf der Oberfläche des Stents ausgebreitet (dispergiert). Eine derartige Verteilung von radioopaken Partikeln bewirkt in der Regel keine ausreichende Dichte dieser Materialien, so dass die Röntgensichtbarkeit für viele Anwendungen zu gering ist.

In der Druckschrift US 6,293,966 B1 wird ein Stent mit radioopaken Markerelementen offenbart, der an seinen distalen bzw. proximalen Enden C-förmig ausgebildete Elemente aufweist, die jeweils eine im Wesentlichen kugelförmige Aufnahme ausbilden. In diese Aufnahmen werden Markerelemente mit kugelförmigen Endabschnitten eingesetzt. Die kugelförmigen Endabschnitte werden formschlüssig und ggf. mittels einer Schweißverbindung in den durch die C-förmigen Elemente gebildeten Aufnahmen befestigt.

Die Druckschrift DE 698 36 656 T2 zeigt und beschreibt einen bioabsorbierbaren Marker mit röntgendichten Bestandteilen zur Verwendung auf einer implantierbaren Endoprothese, wie einen Stent. Die bioabsorbierbaren röntgendichten Marker weisen beispielsweise poröse Abschnitte auf, die mit röntgendichtem Material gefüllt sind. Außerdem wird ein Marker beschrieben, der hohle, hohlraumartige und poröse Abschnitte aufweist, die mit röntgendichtem Material gefüllt sind. Außerdem zeigt der Stand der Technik einen Marker, der als längliches Element wie einem Filament ausgebildet ist, welches um Teile der implantierbaren Endoprothese geschlungen wird.

Druckschrift US 2008/0086195 A1 offenbart eine medizinische Vorrichtung aus einem Metall oder Polymer, welche mittels plasma-elektrolytischer Behandlung mit einer polymerfreien Schicht versehen wird. Gegebenenfalls kann eine metallische Vorbeschichtung aufgebracht werden.

Aus dem Dokument US 2008/0057101 A1 ist eine medizinische Vorrichtung mit einer tragenden Struktur und einem auf der Oberfläche der Struktur angeordneten porösen Körper bekannt, in welchem eine aktive Substanz zur Behandlung oder Vermeidung einer Krankheit vorgesehen ist.

Auch hinsichtlich anderer Anwendungen einer oben angegebenen Vorrichtung, beispielsweise für die Anwendung in Flugzeugen, ist es üblich, verschiedene metallische Materialien miteinander zu kombinieren.

Bei Stents oder anderen Vorrichtungen, deren Grundkörper aus einem metallischen Material bestehen, ergibt sich bei der Anordnung von metallischen Funktionselementen an dem Grundkörper das Problem, dass an dem Kontaktbereich zwischen dem Material des Grundkörpers und dem Material des Funktionselements eine Kontaktkorrosion auftritt. Diese führt zur Zerstörung der Vorrichtung bzw. zur Abtrennung des Funktionselements von dem Grundkörper, so dass die Vorrichtung nicht mehr in der Lage ist, ihre Funktion zu erfüllen, bzw. nicht mehr aufgefunden werden kann. Für das geschilderte Problem enthalten die oben beschriebenen Vorrichtungen aus dem Stand der Technik keine Lösung.

Es ist bekannt, die beschleunigte Korrosion von Implantaten aus Magnesiumlegierungen mit Röntgenmarken durch Komplettbeschichtungen mit polymeren Überzügen zu unterbinden. Dabei kommen sowohl biodegradierbare als auch nichtdegradierbare Polymere zum Einsatz. Ebenfalls denkbar sind Lösungen, in denen der Bereich, in dem sich Röntgenmarker befinden, nur über einen Teil des Grundkörpers vor einem beschleunigten Korrosionsangriff geschützt werden, zum Beispiel durch Tauchen in eine Polymerlösung. Die so beschichteten Implantate zeigen zwar zunächst ein verzögertes Degradationsverhalten, korrodieren allerdings im Moment der Beschädigung des polymeren Überzugs in der gleichen Weise wie ein ungeschütztes Implantat. Eine andere prinzipielle Möglichkeit besteht in der Einbringung von gefüllten Polymeren in die dafür im Implantat vorgesehenen Aussparungen. Der Füllstoff dieser Polymere besteht aus feinen Partikeln von röntgenopaken Elementen. Durch die polymere Umhüllung wird eine Minimierung der realen Kontaktfläche des Markermaterials mit dem Magnesium erreicht und die Lokalelementbindung unterdrückt. Allerdings sind die vom Magnesiumimplantat auf den Röntgenmarker ausgeübte Haltekräfte aufgrund der geringen Festigkeit des Polymers und der Unterschiede in den E-Modulen der beiden Werkstoffe begrenzt. Dies führt nach bereits wenigen Tagen zu einem Verlust der Verbundfestigkeit und zu einer damit verbunden Gefahr des Herauslösens des Röntgenmarkers aus dem Implantat.

Die Aufgabe der vorliegenden Erfindung besteht somit darin, eine Vorrichtung zu schaffen, bei der der direkte metallische Kontakt des Funktionselements zum Grundkörper reduziert und gleichzeitig eine hohe Verbundfestigkeit erzielt wird. Die Aufgabe besteht ferner darin, ein kostengünstiges Verfahren zur Herstellung einer derartigen Vorrichtung anzugeben.

Die obige Aufgabe wird durch eine Vorrichtung gelöst, die zumindest auf- und/oder in dem an die Oberfläche reichenden Grenzbereich zwischen Grundkörper und Funktionselement, wobei das Funktionselement in der Ebene des Grundkörpers befestigt vorliegt, eine auf den Grundkörper auf- und/oder eingebrachte erste Schicht aufweist, welche mittels plasmachemischer Behandlung in einer wässrigen Lösung hergestellt ist, die Phosphationen enthält.

Eine derartige erfindungsgemäße Vorrichtung hat den Vorteil, dass der direkte metallische Kontakt zwischen Funktionselement und Grundkörper reduziert und gleichzeitig eine hohe Verbundfestigkeit erzielt wird. Hierdurch wird die Degradationsdauer einer derartigen Vorrichtung wesentlich verlängert und der Verbleib des Funktionselements an der Vorrichtung auch bei fortgeschrittener Degradation sicher gestellt.

In einem bevorzugten Ausführungsbeispiel weist der Grundkörper mindestens ein zumindest weitestgehend biodegradierbares metallisches Material, vorzugsweise Magnesium oder eine Magnesiumlegierung, auf.

In einem weiteren bevorzugten Ausführungsbeispiel beträgt die Dicke der mittels der plasmachemischen Behandlung auf der Grundkörperoberfläche hergestellten ersten Schicht etwa 1 µm bis etwa 20 µm, vorzugsweise etwa 2 µm bis etwa 8 µm.

In einem weiteren bevorzugten Ausführungsbeispiel ist zumindest in dem an der Oberfläche zugänglichen Grenzbereich zwischen Grundkörper und Funktionselement auf der ersten Schicht eine zweite Schicht mit mindestens einem Polymer aus der Gruppe enthaltend Parylene, Polyurethan und Magnesiumstearat angeordnet.

Hierbei werden als Parylene vollständig lineare, teilkristalline und unvernetzte aromatische Polymere bezeichnet. Diese Polymere lassen sich je nach ihrem Aufbau in vier verschiedene Grundtypen einteilen, nämlich Parylene C, Parylene D, Parylene N und Parylene F. Parylene kann auf das Funktionselement bevorzugt mittels eines Aufdampfprozesses aufgebracht werden.

Das Funktionselement weist vorzugsweise eine oder mehrere röntgen- und/oder radioopake Elemente oder Verbindungen aus der Gruppe bestehend aus Platin, Iridium, Gold, Wolfram, Molybdän, Niob, Tantal, Yttrium, Zirkonium, Ytterbium oder Legierungen aus diesen Metallen auf.

In einem weiteren bevorzugten Ausführungsbeispiel weist die zweite Schicht eine Schichtdicke zwischen etwa 0,5 µm und etwa 5 µm auf.

Die obige Aufgabe wird ferner durch ein Verfahren zur Herstellung einer derartigen Vorrichtung gelöst, mit den folgenden Schritten:
i.) Befestigen eines Funktionselements an dem Grundkörper, das zumindest in einem Teil seines Volumens eine verglichen mit dem Material des Grundkörpers verschiedene metallische Materialzusammensetzung aufweist, die vorzugsweise mindestens teilweise radioopakes und/oder röntgenopakes Material beinhaltet, wobei das Funktionselement in der Ebene des Grundkörpers befestigt vorliegt,
ii.) plasmachemische Behandlung des Grundkörpers mit Funktionselement in einer wässrigen Lösung, die Phosphationen enthält, zur Erzeugung einer ersten Schicht auf mindestens dem Teil der Körperoberfläche der den an die Oberfläche reichenden Grenzbereich zwischen Grundkörper und Funktionselement aufweist/bildet.

Das erfindungsgemäße Verfahren stellt ein kostengünstiges Verfahren zur Herstellung einer Vorrichtung mit obigen Vorteilen dar.

In einem bevorzugten Ausführungsbeispiel wird die erste Schicht nach der plasmachemischen Behandlung in einem Lösungsmittel, vorzugsweise mittels destilliertem H₂O gespült und anschließend vorzugsweise bei einer Temperatur von mindestens 80°C, besonders bevorzugt mindestens etwa 100°C getrocknet, wobei das Trocknen vorzugsweise in einem Umluftofen durchgeführt wird.

In einem bevorzugten Ausführungsbeispiel ist in der wässrigen Lösung ein Puffer, vorzugsweise Kaliumdihydrogenphosphat und/oder Natriumdihydrogenphosphat enthalten. Alternativ oder zusätzlich kann in der wässrigen Lösung auch Calciumdihydrogenphosphat als Puffer enthalten sein, dessen geringe Wasserlöslichkeit durch die Zugabe von Komplexbildnern wie Ethylendiamin erhöht werden kann.

In einem bevorzugten Ausführungsbeispiel des erfindungsgemäßen Verfahrens erfolgt die plasmachemische Behandlung der Körperoberfläche dadurch, dass an den Grundkörper eine gepulste Spannung angelegt wird, deren Amplitude in einem Teil des Behandlungszeitraums eine für das Material des Funktionselement charakteristische Badspannung übersteigt und vorzugsweise im Verlauf der Behandlung ansteigt.

In einem bevorzugten Ausführungsbeispiel wird das Funktionselement mittels Pressen, Nieten, Laserschweißen und/oder Elektronenstrahlschweißen an dem Grundkörper befestigt.

In einem bevorzugten Ausführungsbeispiel beträgt die Stromdichte bei der plasmachemischen Behandlung mindestens etwa 8 mA/cm².

Die erfindungsgemäße Vorrichtung weist ein Schichtsystem, insbesondere auf einem absorbierbaren Magnesium-Implantat auf, das mit als Röntgenmarker wirkenden Funktionselementen aus Wolfram, Tantal, Molybdän, Niob und Zirkon sowie deren Legierungen ausgestattet ist. Dabei wird zunächst der Röntgenmarker mittels bekannter Technologien wie Einpressen, Nieten, Laser- oder Elektronenstrahlschweißen mit dem Grundkörper verbunden. Anschließend erfolgt eine anodische Kontaktierung dieses nunmehr aus einem Verbundwerkstoff bestehenden Bauteils (Vorrichtung). Der Kontaktierungswerkstoff besteht dabei aus einem Aluminium- oder Titan-Draht. Anschließend erfolgt ein Eintauchen in eine wässrige, phosphathaltige Lösung. Nach Anlegen einer gepulsten, stetig steigenden Badspannung, die sich durch lange Pulspausen auszeichnet, kommt es zur Oxidation des Werkstoffs des Grundkörpers und des Funktionselements. Durch die unterschiedlichen elektrochemischen Eigenschaften der Legierung des Grundkörpers einerseits und des Funktionselements andererseits bilden sich auf der Oberfläche beider Elemente unterschiedlich dicke Oxidschichten. An der unmittelbaren Kontaktstelle des Materials des Grundkörpers und des Materials des Funktionselements entsteht eine Grenzfläche zwischen den Oxiden des Grundmaterials und den Oxiden des Funktionselements, das beispielsweise in Form eines Röntgenmarkers vorliegt. Durch die Oxidschichtbildung auf der Oberfläche wird der die Korrosion fördernde unmittelbare Kontakt zwischen dem Material des Grundkörpers und dem Material des Funktionselements auf den innen liegenden Bereich (Innenseite) und somit unter die Oberfläche der Vorrichtung verlagert. Mit weiter steigender Badspannung sind zunächst an der Oberfläche des Grundkörpers, welche vorzugsweise Magnesium aufweist, plasmachemische Effekte zu beobachten, die eine Mischphase aus Oxiden und Phosphaten des Materials des Grundkörpers entstehen lassen. Zur gleichen Zeit wächst die Oxidschicht auf dem Funktionselement weiter. Bei einer für das Material des Funktionselements charakteristischen Badspannung von beispielsweise ca. 230 V für Tantal oder 260 V für Wolfram geht eine durch plasmachemische Effekt bedingte Umwandlung der ursprünglich reinen plasmachemisch erzeugten Oxidschicht in eine Phosphat-/Oxidmischschicht der Funktionselement-Oberfläche vonstatten. Die Dicke der Oxid-/Phosphatmischschicht auf der Funktionselement-Oberfläche wächst dabei abhängig von der Badspannung. Aufgrund der bereits erwähnten unterschiedlichen elektrochemischen Eigenschaften und der damit verbundenen unterschiedlich hohen Sauerstoffaffinität von Grundkörper und Funktionselement muss der plasmachemische Prozess unter anderen Bedingungen ablaufen als die Beschichtung der einzelnen Komponenten. Es ist vor allem von Vorteil, wenn die jeweiligen Oxidschichten langsam wachsen, da andernfalls das Oxid mit der höchsten Bildungsenthalpie keine kohärente Schicht ausbilden kann. Das langsame Wachstum der Oxidschicht wird erfindungsgemäß durch lange Pausen zwischen den Spannungspulsen erreicht. Diese können bis zu 500 Millisekunden lang sein. Dies führt zu einer Rekombination der durch die plasmachemischen Effekte hervorgerufenen Ungleichmäßigkeiten in den Phosphat- und Oxidschichten der Metalle von Grundkörper und Funktionselement.

An der Oberfläche und - je nach Höhe der angelegten Badspannung - bis in eine Tiefe von ca. 10 µm der Grenzfläche zwischen Grundkörper und Funktionselement wird erfindungsgemäß ein gleichmäßiger Übergang der Zusammensetzung erzielt. Durch die plasmachemischen Effekte, die bei höheren Badspannungen sowohl das Material des Grundkörpers als auch das des Funktionselements aufschmelzen und teilweise in die Dampfphase versetzen, erfolgt sowohl eine Durchmischung der Materialien von Grundkörper und Funktionselement als auch eine Durchmischung der entsprechenden Oxide und Phosphate im Bereich der ersten Schicht. Durch das in der wässrigen Lösung (Elektrolyt) des erfindungsgemäßen Verfahrens vorhandene große Sauerstoff- und Phosphorangebot werden in der kurzzeitig stabilen Plasmaphase auch Mischoxide wie zum Beispiel Magnesium-Tantaloxid und Mischphosphate des Materials des Grundkörpers und des Funktionselements gebildet. Diese danach in der Übergangszone (Grenzbereich von Grundkörper und Funktionselement, die sich bis an die Oberfläche der unbehandelten Vorrichtung erstreckt) vorhandenen Verbindungen bilden eine Trennschicht und reduzieren damit den unmittelbaren metallischen Kontakt beider Partner auf Bereiche im Bauteilinneren. Die geringere Korrosionsbeständigkeit dieser innen liegenden Bereiche wirkt sich erst dann aus und beschleunigt entsprechend die Korrosion, wenn die darüberliegenden Oxid- und Phosphatschichten bei Auslagerung unter korrosiven Bedingungen degradiert sind.

Die erfindungsgemäße Oberflächenschicht kann auch als Basisschicht für eine nachfolgende Parylene enthaltende Schicht dienen. Bei einer solchen Schichtkombination wird die Degradationszeit der Vorrichtung nochmals wesentlich erhöht.

Zusätzlich kann der Bereich um das Funktionselement oder die gesamte Vorrichtung mit einem Polymer (Parylene, Polyurethan, Magnesiumstearat) beschichtet werden. Weitere Vorteile sind:
- Es werden Vollmarker verwendet, welche eine hohe Röntgendichte aufweisen (vergleiche Stand der Technik mit im Polymer suspendiertem Material).
- Es wird eine stoffschlüssige oder formschlüssige (Pressen/Nieten) Verbindung zwischen dem Grundkörper und dem Material des Funktionselements ohne Zugabe eines dritten Werkstoffs (wie Polymere oder Klebstoffe) realisiert.
- Es wird ein direkter Metall/Metall-Kontakt von Grundkörper und Funktionselement an der Oberfläche der Vorrichtung vermieden. Hierdurch wird dort die Bildung eines Lokalelements verhindert und die Korrosion gehemmt.
- Beide Metalle werden in einem Prozessschritt passiviert. Eine Montage vorher unterschiedlich behandelter Komponenten und die damit einhergehende Gefahr von Beschädigungen der Oberflächenschicht entfällt.
- Die Breite des technologisch bedingten Spaltes zwischen Funktionselement und Grundkörper wird verringert. Hierdurch verringert sich die Gefahr der Adhäsion von Fremdpartikeln.
- Das erfindungsgemäße Verfahren erzeugt auf beiden Metallen eine verfahrensbedingte poröse Struktur, die eine nachträgliche Versiegelung des Bauteils, z.B. mit polymeren Deckschichten, durch eine bessere Haftung vereinfacht oder als Carrier/Träger (bspw. für eine pharmazeutisch aktive Substanz oder andere funktionelle Stoffe) genutzt werden kann. Hierbei wird unter einer "pharmazeutisch aktiven Substanz" (oder therapeutisch aktive oder wirksame Substanz) ein pflanzlicher, tierischer oder synthetischer Wirkstoff (Medikament) oder ein Hormon verstanden, das in geeigneter Dosierung als Therapeutikum zur Beeinflussung von Zuständen oder Funktionen des Körpers, als Ersatz für natürlich vom Menschen oder tierischen Körper erzeugte Wirkstoffe, wie Insulin, sowie zur Beseitigung oder Unschädlichmachen von Krankheitserregern, Tumoren, Krebszellen oder Körperfremdstoffen Einsatz findet.
- Ferner besteht die Möglichkeit der Beladung der Oberfläche des Funktionselements mit Trägern einer pharmazeutisch aktiven Substanz. Die pharmazeutisch aktiven Substanzen können aufgrund der zum Grundkörper verschiedenen chemischen Zusammensetzung eine andere Freisetzungskinetik entfalten.
- Vorrichtungen aus unterschiedlichen Leichtmetallen und deren Legierungen können nach dem Fügen mittels stoff-, kraft- oder formschlüssiger Prinzipien mit einer Oberflächentechnologie behandelt werden. Hierdurch wird der Einfluss unterschiedlicher Oberflächeneigenschaften verringert, welche bisher eine Einzelbehandlung der Bauteile der Vorrichtung vor dem Zusammensetzen erforderte.
- Die Bildung von Mischoxiden, Mischphosphaten und einfachen Oxiden in der Oberfläche an dem Grenzbereich zwischen Grundkörper und Funktionselement führt zu einer Erhöhung der Korrosionsbeständigkeit dieser sonst aus korrosionstechnischer Schicht kritischen Oberflächenbereiche.

Die Erfindung wird nachfolgend anhand von in Figuren dargestellten Ausführungsbeispielen näher erläutert. Dabei bilden alle beschriebenen und/oder bildlich dargestellten Merkmale den Gegenstand der Erfindung, unabhängig von ihrer Zusammenfassung in den Ansprüchen oder deren Rückbeziehung.

Es zeigen schematisch:
- Fig. 1: einen Querschnitt durch ein erstes Ausführungsbeispiel einer erfindungsgemäßen Vorrichtung,
- Fig. 2: einen vergrößerten Ausschnitt aus Figur 1.

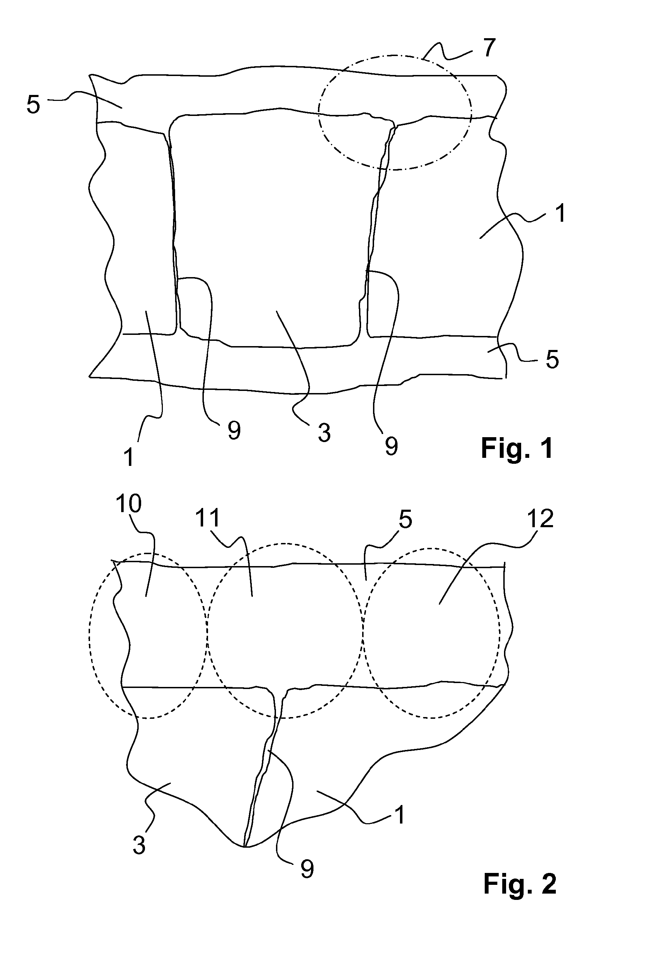

Der in Figur 1 dargestellte Grundkörper 1 einer erfindungsgemäße Vorrichtung in Form eines medizinischen Stents aus der Magnesiumlegierung WE 43 wird mittels Nieten, Elektronenstrahlschweißen oder Laserschweißen mit einem Funktionselement 3 in Form eines Röntgenmarkers aus Wolfram, Tantal, Zirkon oder Niob oder deren Legierung kraft-, form- und/oder stoffschlüssig verbunden. Das so entstandene Verbundelement aus Grundkörper und Funktionselement wird anschließend anodisch kontaktiert und in einen wässrigen Elektrolyt folgender Zusammensetzung (bezogen auf 1 Liter H₂O):
80 g KH₂PO₄
45 g Na₂CO₃
65 ml ED (99%)
5g bis 10 g NaOH
eingetaucht und plasmachemisch oxidiert.

Bei Badendspannungen von 200 bis 500 V und unter Verwendung von gepulsten Strömen mit einem Tastverhältnis von 5 ms on und 100 ms off (im Extremfall bis zu 500 Millisekunden off) und einer Stromdichte von 100 mA/cm², entsteht an der Oberfläche eine erste Schicht 5 aus Oxiden, Mischoxiden, Phosphaten und Mischphosphaten sowie Spinelle der jeweiligen metallischen Grundwerkstoffe. Die einzustellende Badendspannung ist dabei abhängig vom Material des Röntgenmarkers. So wird eine Schichtdicke von 3 µm auf der Magnesiumoberfläche bei einer Schichtdicke von 1,5 µm auf dem Röntgenmarker aus Wolfram bei einer Badenspannung von 260 V erzielt. Nach Erreichen dieser Endspannung fällt die Stromdichte bis auf die Hälfte des ursprünglich eingestellten Wertes ab. Danach wird die Stromzufuhr beendet, der Stent dem Bad entnommen und intensiv unter fließendem destilliertem Wasser gespült und an Warmluft bei ca. 40°C getrocknet. Abschließend wird der Stent sorgfältig vom Kontaktierungsmaterial getrennt und trocken bzw. unter Inertatmosphäre bis zu seiner Weiterverarbeitung gelagert.

Ein Ausschnitt 7 der in Figur 1 gezeigten Struktur ist in Figur 2 vergrößert dargestellt. In der Vergrößerung ist die Fügezone 9, in der das Funktionselement 3 kraftschlüssig und/oder formschlüssig und/oder stoffschlüssig mit dem Grundkörper 1 verbunden/befestigt ist, zu erkennen. In dem Bereich des Übergangs von dem Grundkörper 1 zum Funktionselement 3 ist die Fügezone 9 mit der ersten Schicht 5 bedeckt, welche in den verschiedenen, in Figur 2 mittels gestrichelter Linien umrandeter Zonen bzw. Bereiche der ersten Schicht 5 eine unterschiedliche Zusammensetzung aufweist. In dem über dem Funktionselement 3 liegenden Bereich 10 weist die erste Schicht 5 im Wesentlichen Oxide und/oder Phosphate des Funktionselements 3 auf. Über dem Übergangsbereich zwischen Funktionselement 3 und Grundkörper 1 sind in dem Bereich 11 der ersten Schicht 5 im Wesentlichen sowohl Oxide und/oder Phosphate des Materials des Funktionselements 3 als auch Oxide und/oder Phosphate des Materials des Grundkörpers 1 zu finden. In dem über dem Grundkörper 1 liegenden Bereich 12 der ersten Schicht 5 weist diese im Wesentlichen Oxide und/oder Phosphate des Materials des Grundkörpers 1 auf.

### 3. Beispiel:

Eine erfindungsgemäße Vorrichtung in Form eines Holmes einer tragenden Flügelkonstruktion für Luftfahrzeuge besteht aus einer Titanlegierung TiA16V4, die mittels Laserschweißen mit Spanten aus der Magnesiumknetlegierung AZ 31 verbunden wird. Durch die in Beispiel 1 beschriebene prinzipielle Verfahrensprozedur und den dort verwendeten Elektrolyt erlangt dieser Werkstoffverbund Oberflächeneigenschaften, die einerseits zu einer höheren Korrosionsbeständigkeit führt, andererseits auch ein nachfolgendes Verkleben und/oder Lackieren durch den Wegfall weiterer Vorbehandlungsschritte vereinfacht. Diese aus der Sicht einer Lokalelementbildung kritische Werkstoffkombination - die durch die Bedingungen im Flugzeuginneren wie Schwitzwasser, Taupunktunterschreitung, geringe Luftumwälzung bei langen Standzeiten im Hangar usw. gekennzeichnet ist - kann erst durch die erfindungsgemäße Lösung realisiert werden.

### Bezugszeichenliste

- 1: Grundkörper der erfindungsgemäßen Vorrichtung
- 3: Funktionselement
- 5: erste Schicht
- 7: Ausschnitt
- 9: Übergangsbereich (Fügezone) zwischen Grundkörper 1 und Funktionselement 3
- 10: Bereich der ersten Schicht 5 mit Oxiden und/oder Phosphaten des Grundkörper-Materials
- 11: Bereich der ersten Schicht 5 mit Oxiden und/oder Phosphaten des Grundkörper-Materials und des Funktionselement-Materials
- 12: Bereich der ersten Schicht 5 mit Oxiden und/oder Phosphaten des Funktionselement-Materials

## Patentansprüche

1. Vorrichtung, insbesondere für die medizinische Anwendung als Endoprothese, bevorzugt als intraluminale Endoprothese, mit einem Grundkörper (1), der zumindest teilweise aus einem metallischen Material besteht, und mit einem an dem Grundkörper befestigten Funktionselement (3), das zumindest in einem Teil seines Volumens eine verglichen mit dem Material des Grundkörpers (1) verschiedene metallische Materialzusammensetzung aufweist, die mindestens teilweise radioopakes und/oder röntgenopakes Material aufweist, **dadurch gekennzeichnet, dass** das Funktionselement (3) in der Ebene des Grundkörpers (1) befestigt vorliegt und zumindest auf und/oder in dem an die Oberfläche reichenden Grenzbereich (9) zwischen Grundkörper (1) und Funktionselement (3) eine erste Schicht (5) auf- und/oder eingebracht ist, welche mittels plasmachemischer Behandlung in einer wässrigen Lösung hergestellt ist, die Phosphationen enthält.

2. Vorrichtung nach Anspruch 1, **dadurch gekennzeichnet, dass** der Grundkörper (1) mindestens ein zumindest weitestgehend biodegradierbares metallisches Material, aufweist.

3. Vorrichtung nach Anspruch 2, **dadurch gekennzeichnet, dass** das biodegradierbare Material Magnesium oder eine Magnesiumlegierung, ist.

4. Vorrichtung nach einem der vorhergehenden Ansprüche, **dadurch gekennzeichnet, dass** die Dicke der mittels der plasmachemischen Behandlung auf der Körperoberfläche hergestellten ersten Schicht (5) etwa 1 µm bis etwa 20 µm beträgt.

5. Vorrichtung nach einem der vorhergehenden Ansprüche, **dadurch gekennzeichnet, dass** zumindest in dem an der Oberfläche zugänglichen Grenzbereich (9) zwischen Grundkörper (1) und Funktionselement (3) auf der ersten Schicht (5) eine zweite Schicht mit mindestens einer Verbindung aus der Gruppe enthaltend Parylene, Polyurethan und Magnesiumstearat angeordnet ist.

6. Vorrichtung nach Anspruch 5, **dadurch gekennzeichnet, dass** die zweite Schicht eine Schichtdicke zwischen etwa 0,5 µm und etwa 5 µm aufweist.

7. Vorrichtung nach einem der vorhergehenden Ansprüche, **dadurch gekennzeichnet, dass** das Funktionselement (3) mindestens ein Element oder eine Zusammensetzung aus der Gruppe enthaltend Wolfram, Tantal, Niob, Zirkonium und deren Legierungen aufweist.

8. Verfahren zur Herstellung einer Vorrichtung, die insbesondere als Endoprothese eingesetzt wird, bevorzugt als intraluminale Endoprothese, mit einem Grundkörper (1), der zumindest teilweise aus einem metallischen Material besteht, mit den folgenden Schritten:
i.) Befestigen eines Funktionselements (3) an dem Grundkörper (1), das zumindest in einem Teil seines Volumens eine verglichen mit dem Material des Grundkörpers verschiedene Zusammensetzung aus metallischem Material aufweist, die mindestens teilweise radioopakes und/oder röntgenopakes Material beinhaltet, wobei dass das Funktionselement (3) in der Ebene des Grundkörpers (1) befestigt vorliegt, und
ii.) plasmachemische Behandlung des Grundkörpers (1) mit Funktionselement (3) in einer wässrigen Lösung, die Phosphationen enthält, zur Erzeugung einer ersten Schicht (5) auf mindestens dem Teil der Körperoberfläche, der den an die Oberfläche reichenden Grenzbereich (9) zwischen Grundkörper (1) und Funktionselement (3) bildet.

9. Verfahren nach Anspruch 8, **dadurch gekennzeichnet, dass** die erste Schicht (5) nach der plasmachemischen Behandlung in einem Lösungsmittel, vorzugsweise mittels destilliertem H₂O, gespült und anschließend vorzugsweise bei einer Temperatur von mindestens etwa 80°C getrocknet wird, wobei das Trocknen vorzugsweise in einem Umluftofen durchgeführt wird.

10. Verfahren nach einem der Ansprüche 8 bis 9, **dadurch gekennzeichnet, dass** in der wässrigen Lösung ein Puffer in der Form von Kaliumdihydrogenphosphat und/oder Natriumdihydrogenphosphat und/oder Calciumdihydrogenphosphat enthalten ist.

11. Verfahren nach einem der Ansprüche 8 bis 10, **dadurch gekennzeichnet, dass** die plasmachemische Behandlung der Körperoberfläche **dadurch** erfolgt, dass an den Grundkörper (1) eine gepulste Spannung angelegt wird, deren Amplitude in einem Teil des Behandlungszeitraums eine für das Material des Funktionselements charakteristische Badspannung übersteigt.

12. Verfahren nach einem der Ansprüche 8 bis 11, **dadurch gekennzeichnet, dass** das Funktionselement (3) mittels Pressen oder Nieten oder Laserschweißen oder Elektronenstrahlschweißen an dem Grundkörper (1) befestigt wird.

13. Verfahren nach einem der Ansprüche 8 bis 12, **dadurch gekennzeichnet, dass** die Stromdichte bei der plasmachemischen Behandlung mindestens etwa 8 mA/cm² beträgt.

14. Verfahren nach Anspruch 13, **dadurch gekennzeichnet, dass** die Stromdichte mindestens etwa 10 mA/cm² beträgt.

## Claims

1. A device, in particular for medical use as an endoprosthesis, preferably as an intraluminal endoprosthesis, having a main body (1) consisting at least partially of a metallic material, and having a functional element (3) fastened to the main body, which functional element at least in part of its volume has a metallic material composition that is different in comparison with the material of the main body (1) and that at least partially comprises radiopaque and/or X-ray-opaque material, **characterised in that** the functional element (3) is fastened in the plane of the main body (1) and a first layer (5) at least is applied and/or introduced on and/or in the boundary area (9) that is between the main body (1) and the functional element (3) and extends to the surface, said first layer being produced by means of plasma-chemical treatment in an aqueous solution containing phosphate ions.

2. The device according to Claim 1, **characterised in that** the main body (1) comprises at least one at least largely biodegradable metallic material.

3. The device according to Claim 2, **characterised in that** the biodegradable material is magnesium or a magnesium alloy.

4. The device according to one of the preceding claims, **characterised in that** the thickness of the first layer (5) produced by means of the plasma-chemical treatment on the body surface is approximately 1 µm to approximately 20 µm.

5. The device according to one of the preceding claims, **characterised in that** at least a second layer comprising at least one compound from the group containing parylene, polyurethane and magnesium stearate is arranged in the boundary area (9) between the main body (1) and functional element (3) on the first layer (5), said boundary area (9) being accessible at the surface.

6. The device according to Claim 5, **characterised in that** the second layer has a layer thickness between approximately 0.5 µm and approximately 5 µm.

7. The device according to one of the preceding claims, **characterised in that** the functional element (3) comprises at least one element or a composition selected from the group containing tungsten, tantalum, niobium, zirconium and alloys thereof.

8. A method for producing a device used in particular as an endoprosthesis, preferably as an intraluminal endoprosthesis, having a main body (1) consisting at least partially of a metallic material, comprising the following steps:
i.) fastening a functional element (3) to the main body (1), which functional element has, at least in part of its volume, a metallic material composition that is different in comparison with the material of the main body and that at least partially contains radiopaque and/or X-ray-opaque material, wherein the functional element (3) is fastened in the plane of the main body (1), and
ii.) plasma-chemically treatment of the main body (1) with the functional element (3) in an aqueous solution containing phosphate ions for creating a first layer (5) on at least that part of the body surface which forms the boundary area (9) that is between the main body (1) and the functional element (3) and that extends to the surface.

9. The method according to claim 8, **characterised in that** the first layer (5) is washed in a solvent, preferably by means of distilled H₂O, after the plasma-chemical treatment, and is then dried, preferably at a temperature of at least approximately 80 °C, wherein the drying is preferably performed in a circulating air oven.

10. The method according to one of Claims 8 to 9, **characterised in that** a buffer in the form of potassium dihydrogen phosphate and/or sodium dihydrogen phosphate and/or calcium dihydrogen phosphate is contained in the aqueous solution.

11. The method according to one of Claims 8 to 10, **characterised in that** the plasma-chemical treatment of the body surface is performed by applying to the main body (1) a pulsed voltage with an amplitude exceeding over a part of the treatment time a bath voltage characteristic for the material of the functional element.

12. The method according to one of Claims 8 to 11, **characterised in that** the functional element (3) is fastened to the main body (1) by pressing or riveting or laser welding or electron beam welding.

13. The method according to one of Claims 8 to 12, **characterised in that** the current density in the plasma-chemical treatment is at least approximately 8 mA/cm².

14. The method according to Claim 13, **characterised in that** the current density is at least approximately 10 mA/cm².

## Revendications

1. Dispositif, notamment destiné à un emploi dans le domaine médical en tant qu'endoprothèse, de préférence en tant qu'endoprothèse intraluminale, avec un corps de base (1) qui est au moins partiellement constitué d'un matériau métallique, et avec un élément fonctionnel (3), fixé sur le corps de base, qui présente au moins dans une partie de son volume une composition du matériau métallique différente comparativement au matériau du corps de base (1), qui présente au moins partiellement un matériau radio-opaque et/ou opaque aux rayons X, **caractérisé en ce que** l'élément fonctionnel (3) est présent, fixé dans le plan du corps de base (1), et qu'au moins une première couche (5) est rapportée sur, et/ou est incorporée, dans la zone limitrophe (9) atteignant la surface entre le corps de base (1) et l'élément fonctionnel (3), laquelle est fabriquée au moyen d'un traitement chimique par plasma dans une solution aqueuse qui contient des ions phosphate.

2. Dispositif selon la revendication 1, **caractérisé en ce que** le corps de base (1) présente au moins un matériau métallique biodégradable, au moins en grande partie.

3. Dispositif selon la revendication 2, **caractérisé en ce que** le matériau biodégradable est du magnésium ou un alliage de magnésium.

4. Dispositif selon l'une des revendications précédentes, **caractérisé en ce que** l'épaisseur de la première couche (5) sur la surface du corps fabriquée au moyen du traitement chimique par plasma atteint d'environ 1 µm jusqu'à environ 20 µm.

5. Dispositif selon l'une des revendications précédentes, **caractérisé en ce qu'**au moins une deuxième couche avec au moins un composé faisant partie du groupe contenant le parylène, le polyuréthane et le stéarate de magnésium, est disposée sur la zone limitrophe (9) accessible de la surface entre le corps de base (1) et l'élément fonctionnel (3) de la première couche (5).

6. Dispositif selon la revendication 5, **caractérisé en ce que** la deuxième couche présente une épaisseur de couche entre environ 0,5 µm et environ 5 µm.

7. Dispositif selon l'une des revendications précédentes, **caractérisé en ce que** l'élément fonctionnel (3) présente au moins un élément ou une composition faisant partie du groupe contenant le tungstène, le tantale, le niobium, le zirconium et leurs alliages.

8. Procédé de fabrication d'un dispositif, qui est notamment employé en tant qu'endoprothèse, de préférence en tant qu'endoprothèse intraluminale, avec un corps de base (1) qui est constitué au moins partiellement d'un matériau métallique, avec les étapes suivantes :
i) fixation d'un élément fonctionnel (3) sur le corps de base (1) qui présente au moins dans une partie de son volume une composition de matériau métallique différente comparativement au matériau du corps de base, qui contient au moins partiellement un matériau radio-opaque et/ou opaque aux rayons X, où l'élément fonctionnel (3) est présent fixé dans le plan du corps de base (1), et
ii) traitement chimique par plasma du corps de base (1) avec l'élément fonctionnel 3) dans une solution aqueuse qui contient des ions phosphates, pour la création d'une première couche (5) sur au moins la partie de la surface que forme la zone limitrophe (9) atteignant la surface entre le corps de base (1) et l'élément fonctionnel (3).

9. Procédé selon la revendication 8, **caractérisé en ce que** la première couche (5) est lavée après le traitement chimique par plasma dans un solvant, de préférence, au moyen d'eau distillée H₂O, et est ensuite séchée de préférence à une température d'au moins environ 80 °C, le séchage étant de préférence réalisé dans un four à convection.

10. Procédé selon l'une des revendications 8 à 9, **caractérisé en ce qu'**un tampon sous la forme de dihydrogéno phosphate de potassium, et/ou de dihydrogéno phosphate de sodium, et/ou de dihydrogéno phosphate de calcium est contenu dans la solution aqueuse.

11. Procédé selon l'une des revendications 8 à 10, **caractérisé en ce que** le traitement chimique par plasma de la surface du corps est effectué par le fait qu'une tension alternative, dont l'amplitude dans une partie de la durée de traitement dépasse la tension de bain caractéristique pour le matériau fonctionnel, est appliquée au corps de base (1).

12. Procédé selon l'une des revendications 8 à 11, **caractérisé en ce que** l'élément fonctionnel (3) est fixé sur le corps de base (1) au moyen d'un pressage, ou d'un rivetage, ou d'un soudage par laser, ou d'un soudage par faisceaux d'électrons.

13. Procédé selon l'une des revendications 8 à 12, **caractérisé en ce que** la densité de courant lors du traitement chimique par plasma atteint au moins environ 8 mA/cm².

14. Procédé selon la revendication 13, **caractérisé en ce que** la densité de courant atteint au moins environ 10 mA/cm².
